# EUROPEAN PATENT SPECIFICATION

(11) **EP 1 864 623 B1**
(45) Date of publication and mention of the grant of the patent: **13.09.2017**
(21) Application number: 07011136.4
(22) Date of filing: 06.06.2007
(51) Int. Cl.: A61B 18/14

(54) **Endoscopic treatment instrument and endoscope system**
Endoskopisches Behandlungsinstrument und Endoskopsystem
Instrument de traitement endoscopique et système endoscopique

(30) Priority: 09.06.2006 JP 2006160892
(43) Date of publication of application: 12.12.2007
(73) Proprietor: Olympus Corporation, Tokyo 192-8507 (JP)
(72) Inventor: Okada, Tsutomu, Hachioji-shi, Tokyo 192-8507 (JP)
(74) Representative: von Hellfeld, Axel

(56) References cited:
- JP-A- 2006 223 640
- US-A1- 2003 084 907
- US-A1- 2003 229 341
- US-A1- 2005 228 403
- US-B1- 6 352 503

## Description

### BACKGROUND OF THE INVENTION

### Field of the Invention

The present invention relates to an endoscopic treatment instrument and an endoscope system using such an endoscopic treatment instrument. An endoscopic treatment instrument is known from JP 2006 223640, which represents the most relevant prior art.

### Description of the Related Art

Conventionally, when an endoscopic treatment instrument includes a treating portion with directional constraint such as a snare and the like, there is a case in which when the treating portion is protruded from a distal end of a channel of the endoscope, the treating portion is not oriented in a desired direction (see, for example, Japanese Patent Application, First Publication No. 2005-130965). To respond to this, the endoscopic treatment instrument (inserting portion) is rotated relative to the channel at the proximal side with respect to the operator such that the treating portion is appropriately oriented. However, there is a case in which it is not possible to stop the treating portion in a desired orientation since an inserting portion of the endoscopic treatment instrument is elongated. In view of this, a structure has been proposed in which a treating portion is connected to a distal end of a flat shaped member to thereby restrict a bending direction such that it is possible to restrict the direction in which the treating portion is oriented to coincide with the direction in which the endoscope is bent (see, for example, the specification of West German Utility Model No. 7715649).

### SUMMARY OF THE INVENTION

The above-mentioned JP-A-2006 223640 discloses an endoscopic treatment instrument comprising a connecting member (pipe) and a flat plate as a control rod. The transition between the pipe and the plate is formed by an adhesive or other bonding material.

The object underlying the present invention is to provide an endoscopic treatment instrument, which, however, is easy to manufacture and which ensures smooth operation of the treatment instrument.

The solution to this problem in accordance with the present invention is described in claim 1. Such treatment instrument can be used in an endoscope system in accordance with claim 9.

### BRIEF DESCRIPTION OF THE DRAWINGS

FIG 1 is a plan view illustrating the detail of a high frequency snare of an endoscope system according to a first embodiment of the present invention.
FIG 2 is a general plan view illustrating the high frequency snare according to the first embodiment of the present invention.
FIG 3 is a cross-sectional view illustrating the detail of the high frequency snare according to the first embodiment of the present invention.
FIG 4 is a cross-sectional view illustrating the high frequency share according to the first embodiment as seen from an angle different from FIG 3.
FIG. 5 is an enlarged perspective view illustrating a relevant part of the high frequency snare according to the first embodiment of the present invention.
FIG 6 is a cross-sectional view along line A-A, of FIG 3.
FIG 7 is a plan view illustrating a certain step of conducting an excising operation by the use of the high frequency snare of the endoscope system according to the first embodiment of the present invention.
FIG 8 is a plan view illustrating another step of conducting the excising operation by the use of the high frequency snare of the endoscope system according to the first embodiment of the present invention.
FIG 9 is a perspective view illustrating a part of a high frequency snare according to a second embodiment of the present invention.
FIG 10 is a longitudinal sectional view illustrating an essential: part of the high frequency snare according to the second embodiment of the present invention.
FIG 11 is a longitudinal sectional view illustrating the essential part of the high frequency snare according to the second embodiment of the present invention.
FIG 12 is a disassembled structural view illustrating an essential part of a high frequency snare according to a third embodiment of the present invention.
FIG 13 is a longitudinal sectional view illustrating the essential part of the high frequency snare according to the third embodiment of the present invention.
FIG 14A is a view illustrating a distal end of a control wire of a high frequency snare heretofore used.
FIG. 14B is a view illustrating a distal end of a control wire of the high frequency snare according to the third embodiment of the present invention.
FIG 15A is a longitudinal sectional view illustrating an essential part of a heretofore used high frequency snare in operation.
FIG 15B is a longitudinal sectional view illustrating an essential part of the high frequency snare, in operation, according to the third embodiment of the present invention.
FIG 16A is a perspective view illustrating an essential part of a modified example of the high frequency snare according to the third embodiment of the present invention.
FIG 16B is a side view illustrating the modified example of the high frequency snare depicted in FIG 16A.
FIG 17 is a longitudinal sectional view illustrating the modified example of the high frequency snare in operation according to the third embodiment of the present invention.
FIG 18 is a perspective view illustrating an essential part of a high frequency snare according to a fourth embodiment of the present invention.
FIG 19 is a perspective view illustrating an essential part of a modified example of the high frequency snare according to the fourth embodiment of the present invention.
FIG 20 is a partially perspective view illustrating a certain step of conducting an excising operation by the use of the high frequency snare according to the fourth embodiment of the present invention.
FIG 21 is a partially perspective view illustrating another step of conducting the excising operation by the use of the high frequency snare according to the fourth embodiment of the present invention.
FIG 22 is a perspective view illustrating an essential part of a high frequency snare according to a fifth embodiment of the present invention.
FIG 23 is an illustration when viewed in the direction of arrow B in FIG 22. FIG. 24 is a perspective view illustrating an essential part of a modified example of the high frequency snare according to the fifth embodiment of the present invention.
FIG 25 is a perspective view illustrating an essential part of a further modified example of the high frequency snare according to the fifth embodiment of the present invention.
FIG 26 is a partially perspective view illustrating a certain step of conducting an excising operation by the use of the high frequency snare according 10 the fifth embodiment of the present invention.
FIG 27 is a perspective view illustrating a part of a modified example of the high frequency snare according to the second embodiment of the present invention.
FIG 28 is a longitudinal sectional view illustrating an essential part of the modified example of the high frequency snare according to the second embodiment of the present invention.
FIG 29 is a partially perspective view illustrating an essential part of a modified example of the high frequency snare according to the first embodiment of the present invention.
FIG 30 is a cross-sectional view taken along the arrowed line D-D of FIG 29, of the modified example of the high frequency snare according to the first embodiment of the present invention.
FIG 31 is a cross-sectional view for explaining an operation of the modified example of the high frequency snare according to the first embodiment of the present invention.

### DETAILED DESCRIPTION OF THE INVENTION

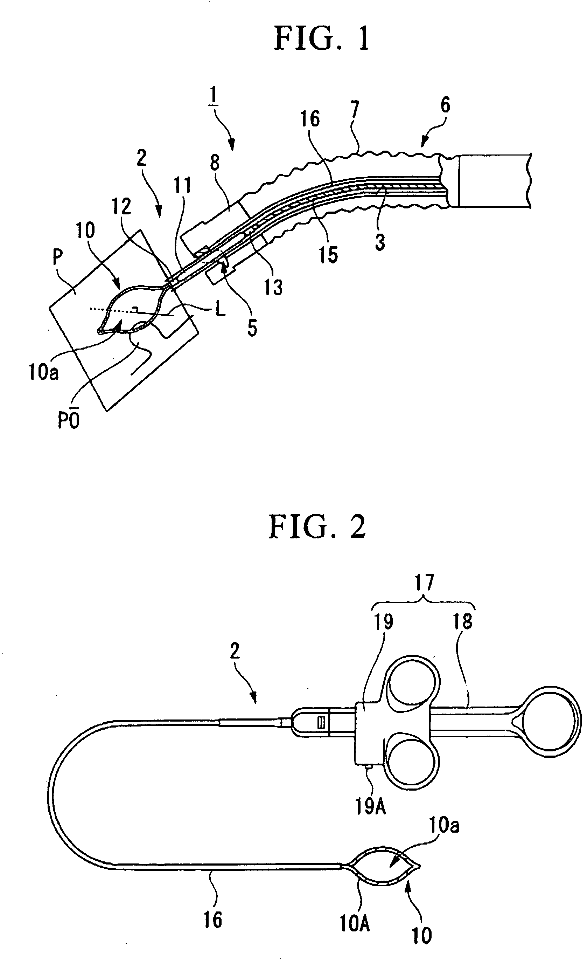

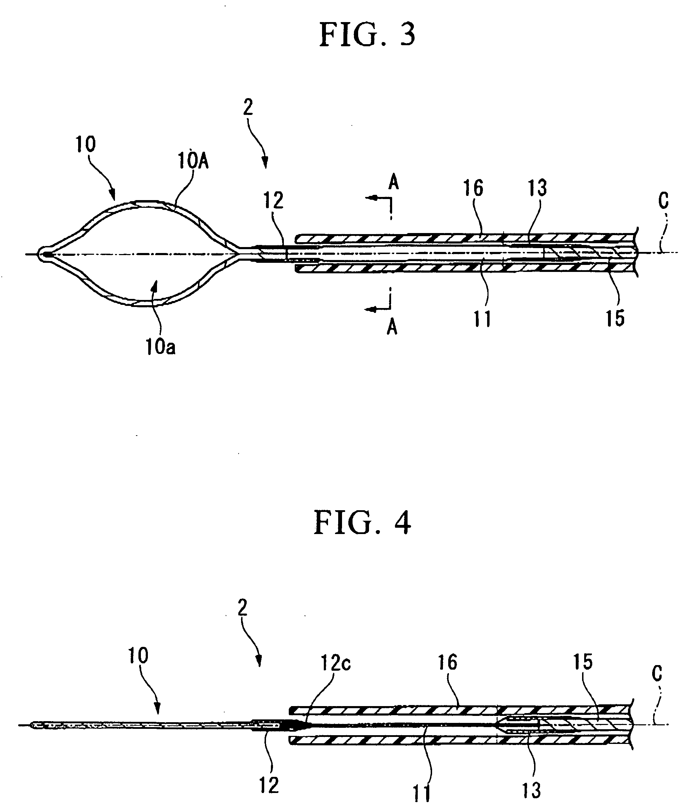

With reference to FIGS. 1 to 8, a description will be given of a first embodiment according to the present invention.

As illustrated in FIG 1, an endoscope system 1 according to the present invention is provided with a high frequency snare (endoscopic treatment instrument) 2 and an endoscope 6. The endoscope 6 includes a channel 3 with a front end opened in which the high frequency snare 2 is inserted so as to retumably protrude from the front end of the channel 3, and a forceps raising unit 5 which is disposed in the vicinity of the front end of the channel 3 and controls a protruding direction of the high frequency snare 2.

On a front end side of the endoscope 6, a bending portion 7 is provided which is bendable by an operation of an unillustrated endoscope operating portion and a distal end rigid portion 8 which is connected to a front end of the bending portion 7 and has the opening of the channel 3. The forceps raising unit 5 is incorporated in the distal end rigid portion 8.

As illustrated in FIGS. 2 to 4, the high frequency snare 2 includes a snare loop (treating portion) 10 for tightly binding a living tissue, a control rod 11 of flat plate shape whose cross-section has a long axis direction and a short axis direction, a distal end side connecting member 12 of cylindrical shape inside of which a proximal end of the snare loop 10 and a distal end of the control rod 11 are mutually, connected, a control wire (control member) 15 which is elongated in an axial direction C of the control rod 11 and connected to the control rod 11 through a proximal end side connecting member 13 of cylindrical shape for applying a drive force of the axial direction C, an elongated sheath 16 which has flexibility and in which the control rod 11 and the snare loop 10 are inserted so as to voluntarily extend and retract (or advance and retreat), and a control portion 17 to which proximal ends of the control wire 15 and the sheath 16 are connected and which extend and retract the control wire 15 with respect to the sheath 16.

The snare loop 10 comprises a dissecting wire 10A made in a loop shape and has a loop plane 10a. Once the snare loop 10 has been received in the sheath 16, the loop plane 10a is in a contracted state.

The control rod 11 which has been formed in a rectangular plate shape is connected to the snare loop 10 in such a manner that a plane defined by the axial direction C of the control rod 11 and by the short axis direction of the control rod cross-section is substantially parallel to the loop plane 10a. That is, the control rod 11 is arranged such that it is easily bendable in the direction of the normal line L of a plane surface P containing the loop plane 10a and is not easily bendable in a direction orthogonal to the normal line L.

The lengths in the long axis and short axis directions of the cross-section of the control rod 11 are such that, in the sheath 16, the control rod 11 is extendable and retractable (or advanceable and retreatable) in the axial direction C and is also voluntarily rotatable. The lengths of a distal end side portion and a proximal end side portion of the control rod 11 in the long axis direction are longer than that of the control rod middle portion so as to make it easier to insert them in the distal end side connecting member 12 and the proximal end side connecting member 13.

The length of the control rod 11 in the axial direction C is set such that the proximal end of the control rod 11 is positioned within the distal end rigid portion 8 when the sheath 16 is inserted in the channel 3 of the endoscope 6 so that a distal end of the control rod 11 is protruded to the fullest extent from the opening of the channel 3. That is, the length is so as not to interfere with the bending portion 7 of the endoscope 7.

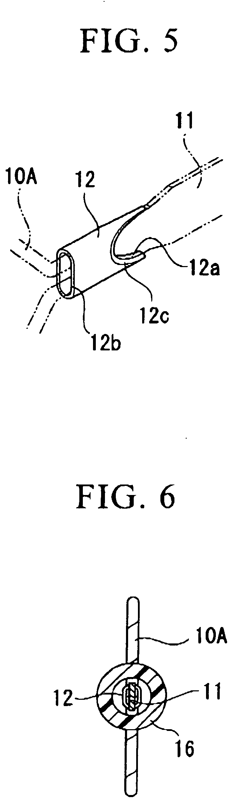

As illustrated in FIGS. 5 and 6, the distal end side connecting member 12 is provided with a first opening end surface 12a and a second opening end surface 12b facing in the axial direction C and is formed into a flat shape with a long axis direction and a short axis direction which correspond to the long axis direction and the short axis direction, respectively, of the cross-section of the control rod 11. The distal end of the control rod 11 is inserted in the first opening end surface 12a and the dissecting wire 10A of the snare loop 10 is inserted in the second opening end surface 12b. The dissecting wire 10A is secured such that two portions thereof are arranged in the long axis direction of the cross-section of the distal end side connecting member 12 and juxtaposed to each other. As illustrated in FIG. 6, the thickness of the control rod 11 is smaller than the outer diameter of the dissecting wire 10A. The short axis of the distal end side connecting member 12 has substantially the same dimension as the outer diameter of the dissecting wire 10A.

The first opening end surface 12a is provided at both sides with inclined surfaces 12c which have a generally flat shape and sandwiches the control rod 11 therebetween. The inclined surfaces 12c intersect the axial direction C of the control rod 11 and the short axis direction of the cross-section of the control rod 11 and are parallel with the long axis direction of the control rod cross-section. In other words; the inclined surfaces 12c are inclined in such a manner that they come close to the axis of the control rod 11 from the middle position between the first opening end surface 12a and the second opening end surface 12b toward the first opening end surface 12a.

The control wire 15 is a stranded wire composed of a bundle of small-gage wires.

As illustrated in FIG 2, the control portion 17 includes a base portion 18 and a slider 19 which is provided to be slidable in front and rear directions with respect to the base portion 18. On the slider 19, an electrode terminal 19A is provided to which a connecting cable for electrical connection with an unillustrated high-frequency power supply is connected. A proximal end of the sheath 16 is connected to the base portion 18 and a proximal end of the control wire 15 is connected to the slider 19. The control wire 15 and the electrode terminal 19A are electrically connected to each other.

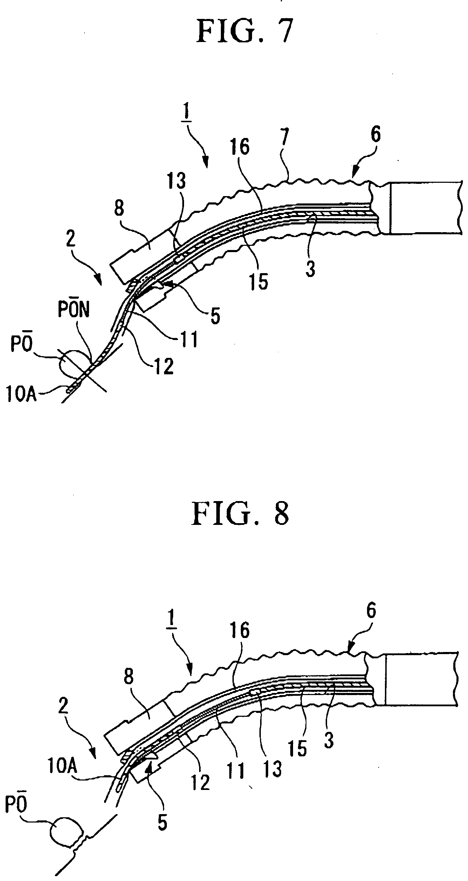

Next, with reference to FIGS. 7 and 8, a description will be given of operation of the high frequency snare 2, as an example, in a case in which a polyp PO is excised within a body cavity by using the endoscope system 1 and the high frequency snare 2 according to the present embodiment.

First, the sheath 16 of the high frequency snare 2 is inserted in the body cavity via the channel 3 of the endoscope 6 such that the sheath 16 protrudes from the front end opening of the channel 3 into the body cavity. Then, the slider 19 of the control portion 17 is advanced relative to the base portion 18. At this time, the control wire 15 is correspondingly advanced, whereby the snare loop 10 is protruded from the sheath 16 through the control rod 11. Then, as illustrated in FIG 1, the snare loop is extended due to a resilient biasing force of the dissecting wire 10A.

Next, the snare loop 10 is positioned so as to hook to or encircle the polyp PO. Specifically, by operating a forceps raising unit 5 of the endoscope 6, the polyp PO is inserted in the snare loop 10 until a neck PON of the polyp PO is encircled by the snare loop 10.

Here, the control rod 11 is positioned at a position where the forceps raising unit 5 of the endoscope 6 is disposed. Therefore, when a direction of the normal line L of the plane surface P containing the loop plane 10a and a protruding direction of the polyp PO coincide with each other, as the control rod 11 is bent in response to motion of the forceps raising unit 5, the snare loop plane 10a accordingly comes close to and goes away from the polyp PO.

On the other hand, when the snare loop 10 is protruded from the sheath 16, since the orientation of the loop plane 10a is variable or undetermined, there are a lot of cases where the direction of the normal line L and the protruding direction of the polyp PO are different from each other.

However, similarly to the case where the direction of the normal line L and the protruding direction of the polyp PO coincide with each other, the forceps raising unit 5 is driven to operate. On this occasion, since the control rod 11 has a rectangular plate shape, a direction of bending thereof is controlled and limited to the plate thickness direction. Accordingly, even if a direction of raising conducted by the forceps raising unit 5 and a direction of a surface of the control rod 11 are different from each other, the control rod 11 is rotated around the axis in response to the raising operation, and finally, the direction of the normal line L of the plane surface P containing the loop plane 10a and the protruding direction of the polyp PO coincide with each other.

Then, as illustrated in FIG 7, the polyp PO is encircled by the snare loop 10, and thereafter, the slider 19 is retracted relative to the base portion 18 of the control portion 17 while the forceps raising unit 5 is being operated for the raising. At this time, the distal end side connecting member 12 passes through a position where the forceps raising unit 5 is disposed.

Here, since the outer diameter of the distal end side connecting member 12 is larger than the plate thickness of the control rod 11, when the distal end side connecting member 12 passes over a position where the forceps raising unit 5 is provided, the forceps raising unit 5 is pressed radially outward of the distal end side connecting member 12 in a direction opposite the raising direction. However, the distal end side connecting member 12 and the forceps raising unit 5 are relatively smoothly moved because the inclined surface 12c is formed on the first opening end surface 12a positioned at the base end side of the distal end side connecting member 12. On the other hand, since the outer diameter of the dissecting wire 10A and the short axis directional (internal) length in the second opening end surface is substantially the same, there is no step (or difference in level) formed, thereby allowing smooth relative movement. Herewith, the snare loop 10 operates or moves and then tightly binds the neck PON of the polyp PO. Thereafter, high frequency current is supplied to the dissecting wire 10A from the unillustrated high-frequency power supply or source to cauterize the neck PON such that the polyp is severed or cut off, as illustrated in FIG 8. Then, the snare loop 10 is received or housed in the sheath 16.

According to the present endoscope system 1 and the high frequency snare 2, since the rectangular plate shaped control rod 11 mentioned above is provided, when the forceps raising unit 5 is operated while the snare loop 10 is protruded from the sheath 16, the control rod 11 is automatically rotated in such a manner that the direction of raising conducted by the forceps raising unit 5 and a short axis direction of the control rod 11 cross-section coincide with each other, so that the control rod 11 can be bent in the direction in line with the raising direction of the forceps raising unit 5.

Further, when the control rod 11 has been connected to the first opening end surface 12a of the distal end side connecting member 12 formed on the inclined surface 12c, there is almost no step for difference in level at a connection section thereof due to the existence of the inclined surface 12c. Thus, for example, when the distal end side connecting member 12 is retreated in the channel 3 of the endoscope 6 provided with the forceps raising unit 5, the distal end side connecting member 12 does not get stuck on the forceps raising unit 5 or can be smoothly displaced. Accordingly, the snare loop 10 can be oriented in a desired direction and predetermined treatment can be performed.

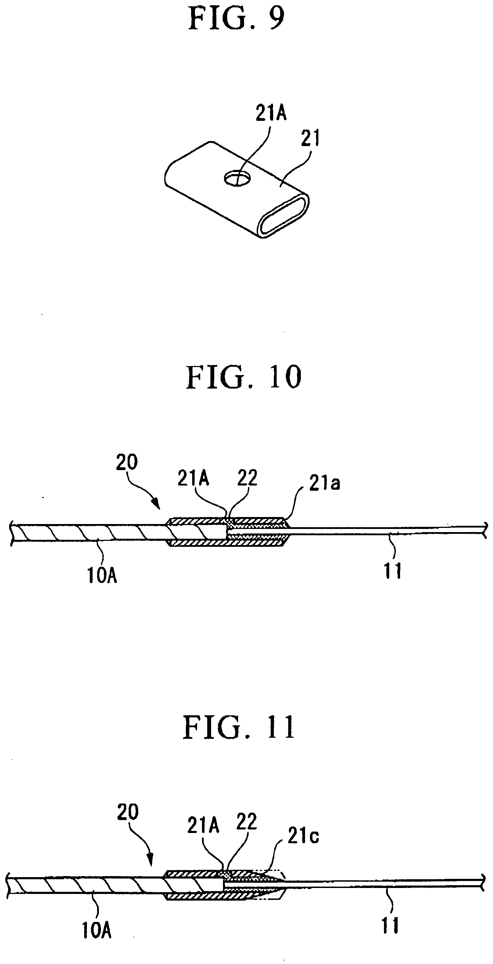

Next, with reference to FIGS. 9 to 11, a description will be given of a second embodiment of the present invention. It should be noted that components which are the same or equivalent as those of the above-mentioned first embodiment are assigned the same reference numerals and that duplicate descriptions are omitted.

The second embodiment is different from the first embodiment in that, instead of previously forming the inclined surface 12c on the distal end side connecting member 12 of the high frequency snare 2 as the first embodiment, a distal end side connecting member 21 of a high frequency snare 20 is connected to the control rod 11 and thereafter an inclined surface 21c is formed.

Specifically, as illustrated in FIG. 9, the distal end side connecting member 21 is formed into an oval pipe shape having a constant cross-sectional area and has at its center an inlet 21A through which a solder (brazing filler metal) 22 is injected therein. As illustrated in FIG 10, through the thus-formed distal end side connecting member 21, the dissecting wire 10A and the control rod 11 are soldered by the solder 22. Afterwards, as illustrated in FIG. 11, part of the first opening end surface 21a is cut to form the inclined surface 21c such as that of the first embodiment. In this way, a high frequency snare 20 having similar shape and structure as those of the second embodiment is formed and obtained.

With this high frequency snare 20, operation and effects that are similar to those of the first embodiment can be achieved.

Especially, since the inclined surface 21c is formed after the dissecting wire 10A and the control rod 11 have been connected to each other, the connection between the control rod 11 and the inclined surface 21c can be made further smooth.

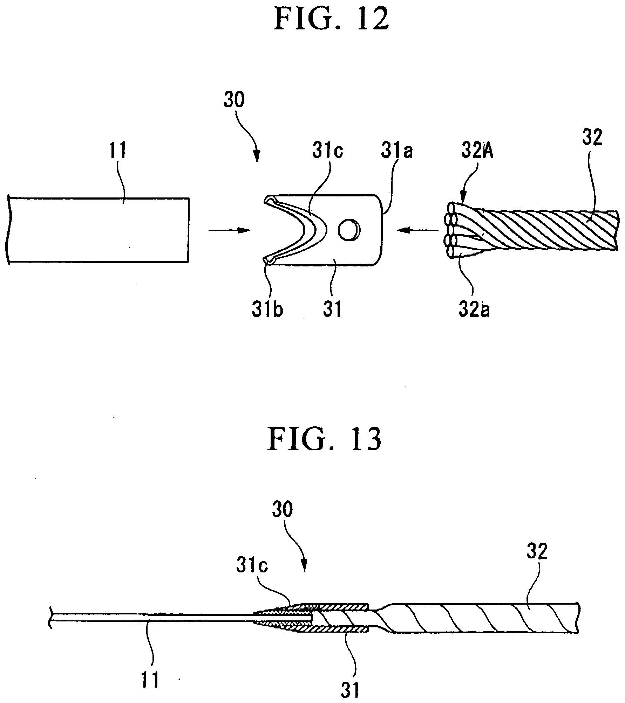

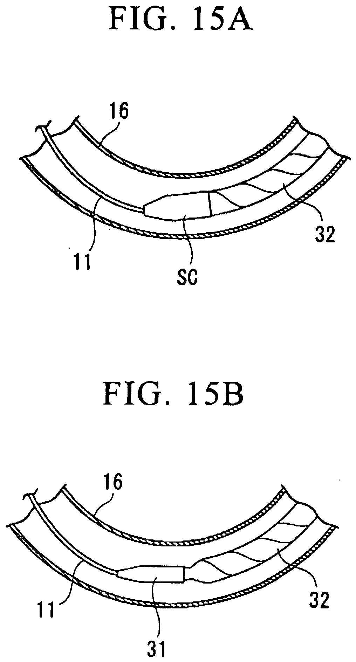

Next, with reference to FIGS. 12 to 15B, a description will be given of a third embodiment of the present invention. It should be noted that components which are the same or equivalent as those of the above-mentioned first and second embodiments are assigned the same reference numerals and that duplicate descriptions are omitted.

The third embodiment is different from the first embodiment in that an inclined surface 31c is formed on a proximal end side connecting member 31 of a high frequency snare 30 according to the present embodiment.

As illustrated in FIG. 12, the proximal end side connecting member 31 is provided with a first opening end surface 31a and a second opening end surface 31b facing in the axial direction C of the control rod 11. The proximal end side connecting member 31 is generally formed into a flat shape with a long axis direction and a short axis direction which are parallel to the long axis direction and the short axis direction, respectively, of the cross-section of the control rod 11. As illustrated in FIG 13, the distal end of a control wire 32 is inserted in the first opening end surface 31a and the proximal end of the control rod 11 is inserted in the second opening end surface 31b.

The second opening end surface 31b is provided at both sides with inclined surfaces 31c which have a generally flat shape and sandwiches the control rod 11 therebetween. The inclined surfaces 31c intersect the axial direction C of the control rod 11 and the short axis direction of the cross-section of the control rod 11 and are parallel with the long axis direction of the control rod cross-section. In other words, the inclined surfaces 31c are inclined in such a manner that they come close to the axis of the control rod 11 from the middle position between the first opening end surface 31a and the second opening end surface 31b toward the first opening end surface 31a.

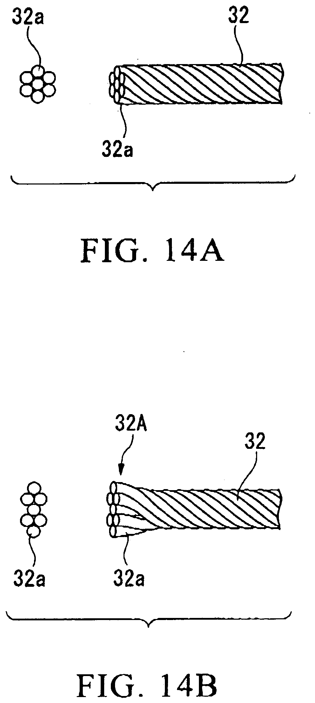

A flat portion 32A which is insertable in the proximal end side connecting member 31 through the first opening end surface 31a is formed on the tip of the control wire 32. The flat portion 32A of the control wire 32 (which is a stranded wire composed of a bundle of small-gage wires 32a) is deformed and collapsed by squeezing such that it is changed from the state illustrated in FIG. 14A to the state illustrated in FIG. 14B so as to be received in the proximal end side connecting member 31 through the first opening end surface 31a. The control rod 11 and the control wire 32 are soldered in a manner similar to the second embodiment.

Here, a description will be given of an operation of a high frequency snare 30 of the present embodiment.

First, similarly to the first embodiment, an illustrated snare loop is set so as to encircle an unillustrated polyp, and then, a slider is retracted toward a base of a control portion (not illustrated) while a forceps raising unit (not illustrated) is being operated or in a raised state. At this time, the proximal end side connecting member 31 passes over a bent portion of the sheath 16.

At this stage, the control wire 32 and the control rod 11 (only in a plate thickness direction thereof) are bent toward a bending direction of the sheath 16. However, the proximal end side connecting member 31 is generally difficult to be bent due to rigidity thereof. Accordingly, a heretofore used, proximal end side connecting member SC such as illustrated in FIG 15A is difficult to pass through the bent portion of the sheath 16. On the other hand, in the high frequency snare 30 according to the present embodiment, when the proximal end side connecting member 31 passes through the bent portion of the sheath, the proximal end side connecting member 31 is rotated in the axial direction relative to the sheath 16 such that a cross-sectional short axis direction thereof coincides with the bending direction, so that the orientation of proximal end side connecting member 31 is controlled. Thus, the proximal end side connecting member is smoothly displaced. In this way, a polyp is excised and then the snare loop 10 is received or housed in the sheath 16.

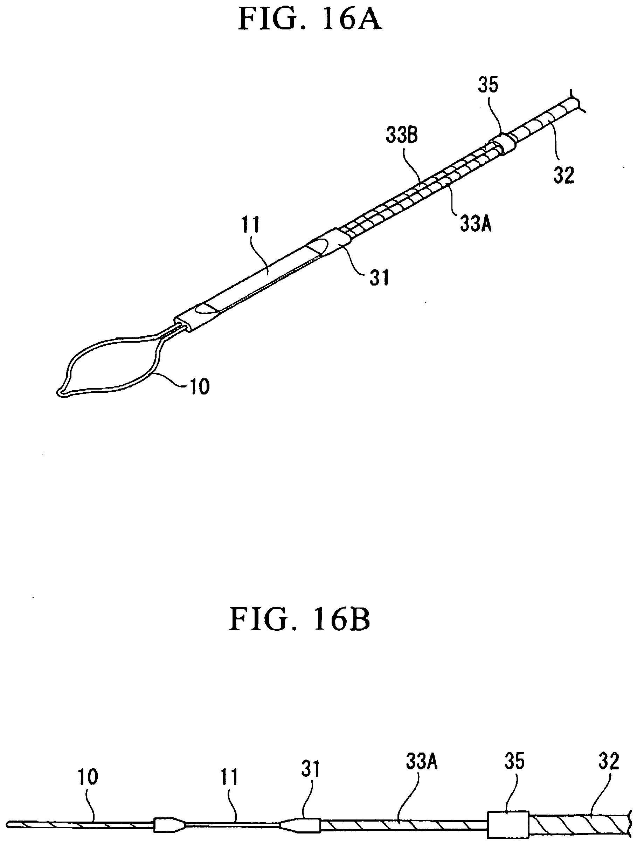

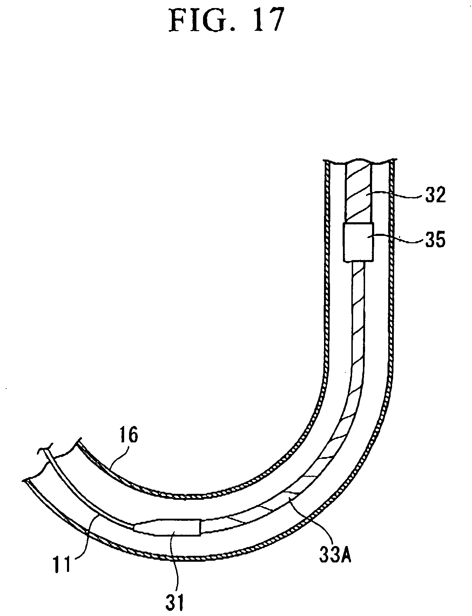

With this high frequency snare 30, when the proximal end side connecting member 31 passes through the bent portion of the sheath 16, it is possible to make the cross-sectional short axis direction of the proximal end side connecting member 31 coincide with the bending direction and to thereby reduce resistance from relating portions thereof so that a smooth displacement is possible. Note that, alternatively, as illustrated in FIG 16A and 16B, wire portions 33A and 33B that are parallel to each other may be provided between the proximal end side connecting member 31 and the control wire 32, and back ends of the wire portions 33A and 33B and a front end of the control wire 32 may be connected through a rear end connecting portion 35. In this alternative, as illustrated in FIG. 17, the rear end connecting portion, 35 is provided such that it is not positioned within the bent portion of the sheath 16 in operation.

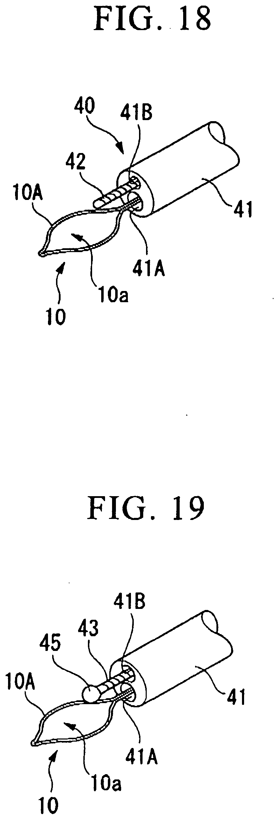

Next, with reference to FIGS. 18 to 21, a description will be given of a fourth embodiment of the present invention. It should be noted that components which are the same or equivalent as those of the above-mentioned embodiments are assigned the same reference numerals and that duplicate descriptions are omitted.

The fourth embodiment is different from the first embodiment in that a push rod 42 is further provided by means of which a snare 40 is moved back and forth relative to a sheath 41 in the same direction as the snare loop 10.

The sheath 41 is provided with a first lumen 41A in which the snare loop 10 is voluntarily inserted and a second lumen 41B in which the push rod 42 is voluntarily inserted. Note that, alternatively, as illustrated in FIG 19, to maintain a contact area to a polyp and the like, a push rod 43 with a large diameter portion 45 may be provided in which the large diameter portion 45 is provided at a tip end of the push rod 43 and has a diameter larger than that of the push rod 43.

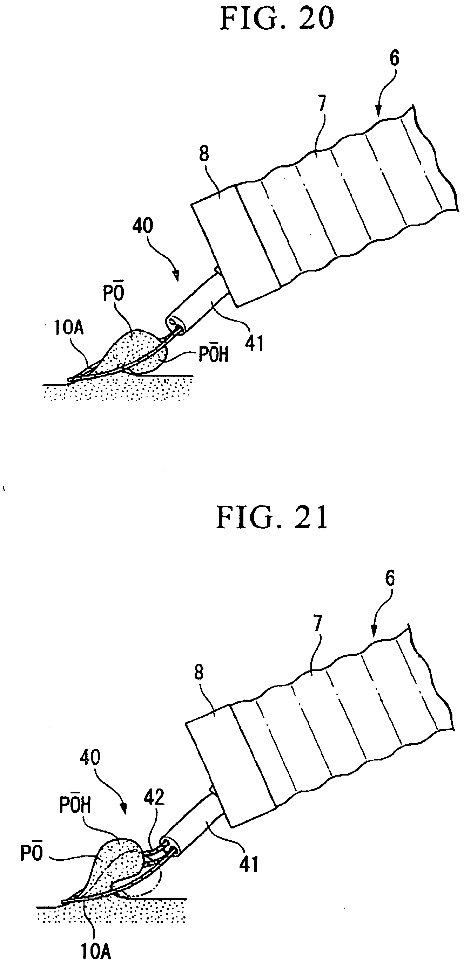

Here, with reference to FIGS. 20 and 21, a description will be given of an operation of a high frequency snare 40 of the present embodiment.

First, similarly to the first embodiment, a front end of the sheath 41 is protruded from a channel opening of the endoscope 6 (not illustrated) and the snare loop 10 is then protruded from the sheath 41.

Then, by an operation of the endoscope, a polyp PO is inserted in the loop plane 10a. At this time, when a head portion POH of the polyp PO is drooping as illustrated in FIG 20, a neck portion PON of the polyp PO cannot be successfully encircled by the dissecting wire 10A, if nothing else is done.

To settle this, the push rod 42 is protruded from the second lumen 41B of the sheath 41 such that the head portion POH of the polyp PO is turned up or raised as illustrated in FIG 21. Thereby, the neck portion PON of the polyp PO is revealed or exposed circumferentially. On this occasion, the dissecting wire 10A or the snare loop 10 is set so as to loosely fit on the polyp PO or encircle the neck portion PON thereof. Thereafter, by an operation similar to that described in the first embodiment, the polyp is excised or cut therefrom.

With this high frequency snare 40, even in the case in which a head POH of a polyp PO is bent down or drooping, an appropriate excision of the polyp is possible.

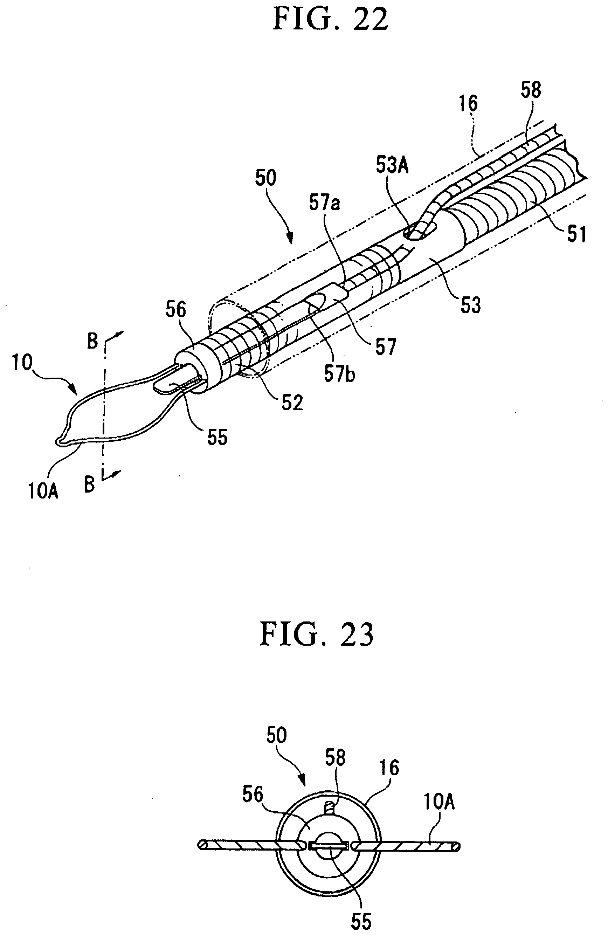

Next, with reference to FIGS. 22 to 26, a description will be given of a fifth embodiment of the present invention. It should be noted that components which are the same or equivalent as those of the above-mentioned embodiments are assigned the same reference numerals and that duplicate descriptions are omitted.

The fifth embodiment is different from the forth embodiment in that the snare loop 10 of a high frequency snare 50 according to the present embodiment is connected to a tip end side of a control tube 52 provided on a front end of a control wire 51.

As illustrated in FIGS. 22 and 23, the control wire 51 and the control tube 52 have substantially the same outer diameter and are connected through a connecting tube 53. In the control tube 52, a push plate 55 which has a rectangular plate shape is extendably and retractably inserted. To a tip end of the control tube 52 is connected a regulating member 56 which regulates rotation of the snare loop 10 and the push plate 55 such that the loop plane 10a of the snare loop 10 and a plate thickness direction of the push plate 55 are maintained to be substantially parallel to each other. The connecting tube 53 is formed with a side opening 53A through which a plate driving wire 58 described below is inserted.

The plate driving wire 58 is connected to a proximal end of the push plate 55 and advances and retreats the push plate 55 relative to the control tube 52 via a generally cylindrical connecting member 57. The plate driving wire 58 and the control wire 51 are disposed in a juxtaposed manner within the sheath 16 such that the plate driving wire 58 is extendable and retractable with respect to the control wire 51. The connecting member 57 is formed into a shape similar to the proximal end side connecting member 31 according to the third embodiment and has a first opening end surface 57a to which a distal end of the plate driving wire 58 is connected and a second opening end surface 57b to which a proximal end of the push plate 55 is connected.

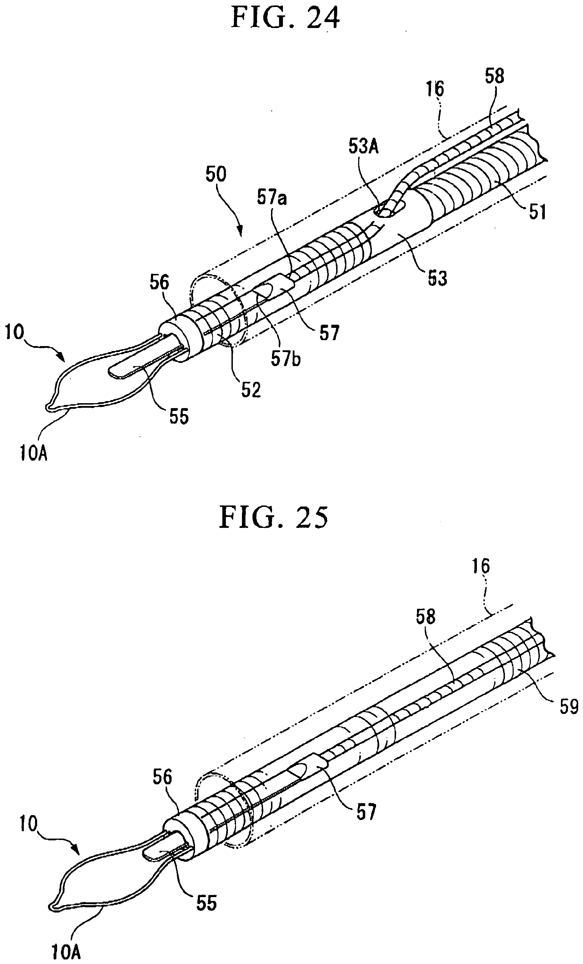

Note that, alternatively, as illustrated in FIG 24, a control tube 59 which is elongated to the vicinity of an operator and in which the plate driving wire 58 is disposed so as to be extendable and retractable may be provided.

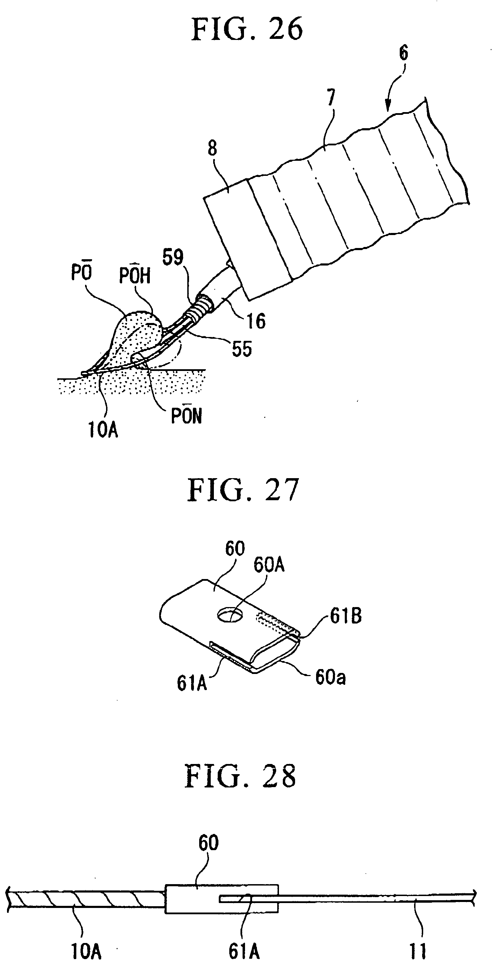

Here, with reference to FIGS. 25 and 26, a description will be given of an operation of a high frequency snare 50 of the present embodiment.

Similarly to the fourth embodiment, the sheath 16 front end is protruded from a channel opening of the endoscope 6 (not illustrated) and the snare loop 10 is then protruded from the sheath 16 so as to encircle a polyp PO. At this time, when a head portion POH of the polyp PO is bent down or drooping, as illustrated in FIG 25, the plate driving wire 58 is advanced relative to the control tube 59 so that the push plate 55 is protruded from the distal end of the control tube 59. As illustrated in FIG 26, the head portion POH of the polyp PO is thereby turned up or raised. Then, the dissecting wire 10A or the snare loop 10 is set so as to loosely fit on the polyp PO or encircle the neck portion PON of the polyp PO. Thereafter, by an operation similar to that described in the first embodiment, the polyp is excised or cut therefrom. With this high frequency snare 50, it is possible to achieve an effect similar to that of the fourth embodiment.

The invention is not limited to the present embodiments illustrated and described herein. However, it should be understood that various changes and modifications may be made therein without departing from the scope of the present invention.

For example, in the second embodiment described above, the distal end side connecting member 21 is formed into an oval pipe shape (or flat cylindrical shape) and provided with the inlet 21A through which a solder is injected therein. Alternatively, as illustrated in FIG. 27, a distal end side connecting member 60 may be provided having an inlet 60A and a pair of slits 61A and 61B extending in parallel to each other from a first opening end surface 60a along the length of the distal end side connecting member 60.

The inside dimension of each of the slits 61A and 61b is substantially equal to the plate thickness of the control rod 11 such that opposed edge portions of the control rod 11 are fittable in the slits 61A and 61B.

Therefore, as illustrated in FIG 28, by connecting the distal end side connecting member 60 and the control rod 11, the relative positioning thereof can be further assured.

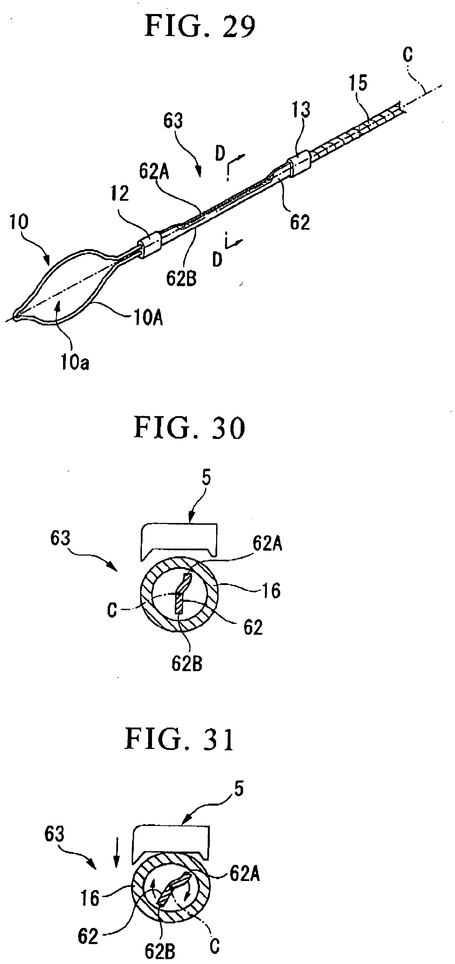

Further, as illustrated in FIGS. 29, a high frequency snare 63 may alternatively be provided in which one half side portion 62A of a control rod 62 is bent at an obtuse angle to the other half side portion 62B of the control rod 62, as can be seen from FIG. 30.

As illustrated in FIG 30, in a state in which the high frequency snare 63 is disposed such that a direction from the axis C toward the other half side portion 62B coincides with a raising direction conducted by the forceps raising unit 5, the forceps raising unit 5 is operated. At this time, since the raising direction of the forceps raising unit 5 intersects a direction from the axis C toward the one half side portion 62A, as illustrated in FIG. 31, a rotation torque around the axis C is generated on the control rod 11.

The forceps raising unit 5 is further raised, and thereby, the control rod 62 is rotated until the raising direction and a direction of the normal line of the control rod 62 become substantially parallel to each other, so that the control rod 62 is bent toward the raising direction. As described above, with this high frequency snare 63, even if the raising direction conducted by the forceps raising unit 5 and the extending direction of the other half side portion 62B are parallel to each other, it is possible to bend the control rod 62.

While preferred embodiments of the invention have been described and illustrated above, it should be understood that these are exemplary of the invention and are not to be considered as limiting. Additions, omissions, substitutions, and other modifications can be made without departing from the scope of the present invention. Accordingly, the invention is not to be considered as being limited by the foregoing description, and is only limited by the scope of the appended claims.

## Claims

1. An endoscopic treatment instrument comprising:
a treating portion (10) for carrying out a predetermined treatment with respect to a body;
a control rod (11) of flat plate shape with a cross-section having a long axis direction and a short axis direction; and
a distal end side connecting member (21), formed into an oval pipe shape having a constant cross-sectional area and having a proximal opening end surface and a distal opening end surface facing in the axial direction (C) of the control rod (11), in which a distal end of the control rod (11) and a dissection wire (10A) are respectively inserted, and through which the dissection wire (10A) and the control rod (11) are connected; wherein the distal end side connecting member (21) has an inlet (21A) at its center, through which a solder (22) has been injected, the solder connecting the dissection wire (10A) and the control rod (11) by means of soldering, and
the proximal opening end surface (21a) of said distal end side connecting member (21) is formed with an inclined surface (21c) of generally flat shape which intersects the axial direction (C) of the control rod (11) and the short axis direction of the cross-section of the control rod (11) and is parallel with the long axis direction of the cross-section of the control rod (11).

2. The endoscopic treatment instrument as recited in claim 1, wherein either of the first opening end surface and the second opening end surface of the distal end side connecting member is connected to the control rod and has a flat shape with a long axis direction and a short axis direction.

3. The endoscopic treatment instrument as recited in claim 1, wherein the distal end side connecting member is of flat shape with a cross-section having a long axis direction and a short axis direction.

4. The endoscopic treatment instrument as recited in claim 1, wherein the treating portion (10) is a snare loop to bind the tissue of the body.

5. The endoscopic treatment instrument as recited in claim 1, further comprising:
a control member (15; 32; 51) which is elongated in the axial direction (C) of the control rod (11) and applies a drive force in the axial direction (C); and
a proximal end side connecting member (13; 31) in which a distal end of the control member (15; 32; 51) and a proximal end of the control rod (11) are both inserted and through which the control member (15; 32; 51) and the control rod (11) are connected;
wherein the proximal end side connecting member (13; 31) is provided with a first opening end surface (31a) and a second opening end surface (31b) in the axial direction (C) of the control rod (11) and
at least one of the first opening end surface (31a) and the second opening end surface (31b) of said proximal end side connecting member (13; 31) is formed with an inclined surface (31c) of generally flat shape which intersects the axial direction (C) of the control rod (11) and the short axis direction of the cross-section of the control rod (11) and is parallel with the long axis direction of the cross-section of the control rod (11).

6. The endoscopic treatment instrument as recited in claim 5, wherein either of the first opening end surface (31a) and the second opening end surface (31b) of the proximal end side connecting member is connected to the control rod and has a flat shape with a long axis direction and a short axis direction.

7. The endoscopic treatment instrument as recited in claim 5, wherein the proximal end side connecting member is of flat shape with a cross-section having a long axis direction and a short axis direction.

8. The endoscopic treatment instrument as recited in claim 5, wherein the treating portion (10) is a snare loop to bind the tissue of the body.

9. An endoscope system comprising:
an endoscopic treatment instrument as recited in claim 1; and
an endoscope including a channel (3) which has an opening at its distal end
and
in which the endoscopic treatment instrument is received so as to extend and retract through the distal end opening, and a forceps raising unit (5) which is disposed in the vicinity of the distal end of the channel (3) and controls a protruding direction of the endoscopic treatment instrument.

10. An endoscope system comprising:
an endoscopic treatment instrument as recited in claim 5; and
an endoscope including a channel (3) which has an opening at its distal end and in which the endoscopic treatment instrument is received so as to extend and retract through the distal end opening, and a forceps raising unit (5) which is disposed in the vicinity of the distal end of the channel (3) and controls a protruding direction of the endoscopic treatment instrument.

## Patentansprüche

1. Endoskopisches Behandlungsinstrument, das umfasst:
einen Behandlungsabschnitt (10) zum Durchführen einer vordefinierten Behandlung in Bezug auf einen Körper;
eine Steuerstange (11) in Form einer flachen Platte mit einem Querschnitt mit einer Langachsenrichtung und einer Kurzachsenrichtung; und
ein distalendseitiges Verbindungselement (21), das in eine ovale Rohrform mit einer konstanten Querschnittsfläche geformt ist und eine proximale Öffnungsendfläche und eine distale Öffnungsendfläche aufweist, die in die Axialrichtung (C) der Steuerstange (11) gerichtet sind, in die ein distales Ende der Steuerstange (11) und ein Sezierdraht (10A) jeweils eingeführt sind und durch die der Sezierdraht (10A) und die Steuerstange (11) verbunden sind;
wobei
das distalendseitige Verbindungselement (21) einen Einlass (21A) in seiner Mitte aufweist, durch den ein Lot (22) gespritzt wurde, wobei das Lot den Sezierdraht (10A) und die Steuerstange (11) durch Löten verbindet, und
die proximale Öffnungsendfläche (21a) des distalendseitigen Verbindungselements (21) mit einer geneigten Oberfläche (21c) mit einer im Allgemeinen flachen Form gebildet ist, die in Axialrichtung (C) der Steuerstange (11) und der Kurzachsenrichtung des Querschnitts der Steuerstange (11) schneidet und parallel zur Langachsenrichtung des Querschnitts der Steuerstange (11) verläuft.

2. Endoskopisches Behandlungsinstrument nach Anspruch 1, wobei eine der ersten Öffnungsendfläche und der zweiten Öffnungsendfläche des distalendseitigen Verbindungselements mit der Steuerstange verbunden ist und eine flache Form mit einer Langachsenrichtung und einer Kurzachsenrichtung aufweist.

3. Endoskopisches Behandlungsinstrument nach Anspruch 1, wobei das distalendseitige Verbindungselement eine flache Form mit einem Querschnitt mit einer Langachsenrichtung und einer Kurzachsenrichtung aufweist.

4. Endoskopisches Behandlungsinstrument nach Anspruch 1, wobei der Behandlungsabschnitt (10) eine Schlingenschleife ist, um das Gewebe des Körpers zu binden.

5. Endoskopisches Behandlungsinstrument nach Anspruch 1, das ferner umfasst:
ein Steuerelement (15; 32; 51), das sich in Axialrichtung (C) der Steuerstange (11) längserstreckt und eine Antriebskraft in Axialrichtung (C) ausübt; und
ein proximalendseitiges Verbindungselement (13; 31), in das ein distales Ende des Steuerelements (15; 32; 51) und ein proximales Ende der Steuerstange (11) jeweils eingeführt sind und durch das das Steuerelement (15; 32; 51) und die Steuerstange (11) verbunden sind;
wobei das proximalendseitige Verbindungselement (13; 31) mit einer ersten Öffnungsendfläche (31a) und einer zweiten Öffnungsendfläche (31b) in Axialrichtung (C) der Steuerstange (11) versehen ist, und
zumindest eine der ersten Öffnungsendfläche (31a) und der zweiten Öffnungsendfläche (31b) des proximalendseitigen Verbindungselements (13; 31) mit einer geneigten Oberfläche (31c) mit einer im Allgemeinen flachen Form gebildet ist, die die Axialrichtung (C) der Steuerstange (11) und die Kurzachsenrichtung des Querschnitts der Steuerstange (11) schneidet und parallel zur Langachsenrichtung des Querschnitts der Steuerstange (11) verläuft.

6. Endoskopisches Behandlungsinstrument nach Anspruch 5, wobei eine der ersten Öffnungsendfläche (31a) und der zweiten Öffnungsendfläche (31b) des proximalendseitigen Verbindungselements mit der Steuerstange verbunden ist und eine flache Form mit einer Langachsenrichtung und einer Kurzachsenrichtung aufweist.

7. Endoskopisches Behandlungsinstrument nach Anspruch 5, wobei das proximalendseitige Verbindungselement eine flache Form mit einem Querschnitt mit einer Langachsenrichtung und einer Kurzachsenrichtung aufweist.

8. Endoskopisches Behandlungsinstrument nach Anspruch 5, wobei der Behandlungsabschnitt (10) eine Schlingenschleife ist, um das Gewebe des Körpers zu binden.

9. Endoskopsystem, das umfasst:
ein endoskopisches Behandlungsinstrument nach Anspruch 1; und
ein Endoskop, das einen Kanal (3) umfasst, der eine Öffnung an seinem distalen Ende aufweist, und
wobei das endoskopische Behandlungsinstrument so aufgenommen wird, dass es sich durch die distale Endöffnung erweitert und zurückzieht, und eine Pinzettenhebeeinheit (5), die in der Nähe des distalen Endes des Kanals (3) angeordnet ist und eine Vorsprungsrichtung des endoskopischen Behandlungsinstruments steuert.

10. Endoskopsystem, das umfasst:
ein endoskopisches Behandlungsinstrument nach Anspruch 5; und
ein Endoskop, das einen Kanal (3) umfasst, der eine Öffnung an seinem distalen Ende aufweist,
und wobei das endoskopische Behandlungsinstrument so aufgenommen wird, dass es sich durch die distale Endöffnung erweitert und zurückzieht, und eine Pinzettenhebeeinheit (5), die in der Nähe des distalen Endes des Kanals (3) angeordnet ist und eine Vorsprungsrichtung des endoskopischen Behandlungsinstruments steuert.

## Revendications

1. Instrument de traitement endoscopique comprenant :
une partie de traitement (10) pour réaliser un traitement prédéterminé par rapport à un corps ;
une tige de commande (11) en forme de plaque plate avec une section transversale ayant une direction de grand axe et une direction de petit axe ; et
un élément de liaison côté extrémité distale (21), configuré en une forme de tuyau ovale ayant une aire en coupe transversale constante et ayant une surface d'extrémité d'ouverture proximale et une surface d'extrémité d'ouverture distale orientées dans la direction axiale (C) de la tige de commande (11), dans lequel une extrémité distale de la tige de commande (11) et un fil de dissection (10A) sont respectivement introduits, et à travers lequel le fil de dissection (10A) et la tige de commande (11) sont reliés ;
l'élément de liaison côté extrémité distale (21) ayant une entrée (21A) à son centre, à travers laquelle une soudure (22) a été injectée, la soudure reliant le fil de dissection (10A) et la tige de commande (11) au moyen d'un soudage, et
la surface d'extrémité d'ouverture proximale (21a) dudit élément de liaison côté extrémité distale (21) comportant une surface inclinée (21c) de forme généralement plate qui coupe la direction axiale (C) de la tige de commande (11) et la direction de petit axe de la section transversale de la tige de commande (11) et est parallèle à la direction de grand axe de la section transversale de la tige de commande (11).

2. Instrument de traitement endoscopique selon la revendication 1, dans lequel l'une ou l'autre de la première surface d'extrémité d'ouverture et de la seconde surface d'extrémité d'ouverture de l'élément de liaison côté extrémité distale est reliée à la tige de commande et a une forme plate avec une direction de grand axe et une direction de petit axe.

3. Instrument de traitement endoscopique selon la revendication 1, dans lequel l'élément de liaison côté extrémité distale a une forme plate avec une section transversale ayant une direction de grand axe et une direction de petit axe.

4. Instrument de traitement endoscopique selon la revendication 1, dans lequel la partie de traitement (10) est une boucle d'anse pour lier le tissu du corps.

5. Instrument de traitement endoscopique selon la revendication 1, comprenant en outre :
un élément de commande (15 ; 32 ; 51) qui est allongé dans la direction axiale (C) de la tige de commande (11) et applique une force d'entraînement dans la direction axiale (C) ; et
un élément de liaison côté extrémité proximale (13 ; 31) dans lequel une extrémité distale de l'élément de commande (15 ; 32 ; 51) et une extrémité proximale de la tige de commande (11) sont toutes deux introduites, et à travers lequel l'élément de commande (15 ; 32 ; 51) et la tige de commande (11) sont reliés ;
l'élément de liaison côté extrémité proximale (13 ; 31) comportant une première surface d'extrémité d'ouverture (31a) et une seconde surface d'extrémité d'ouverture (31b) dans la direction axiale (C) de la tige de commande (11), et
au moins une de la première surface d'extrémité d'ouverture (31a) et de la seconde surface d'extrémité d'ouverture (31b) dudit élément de liaison côté extrémité proximale (13 ; 31) comportant une surface inclinée (31c) de forme généralement plate qui coupe la direction axiale (C) de la tige de commande (11) et la direction de petit axe de la section transversale de la tige de commande (11) et est parallèle à la direction de grand axe de la section transversale de la tige de commande (11).

6. Instrument de traitement endoscopique selon la revendication 5, dans lequel l'une ou l'autre de la première surface d'extrémité d'ouverture (31a) et de la seconde surface d'extrémité d'ouverture (31b) de l'élément de liaison côté extrémité proximale est reliée à la tige de commande et a une forme plate avec une direction de grand axe et une direction de petit axe.

7. Instrument de traitement endoscopique selon la revendication 5, dans lequel l'élément de liaison côté extrémité proximale est de forme plate avec une section transversale ayant une direction de grand axe et une direction de petit axe.

8. Instrument de traitement endoscopique selon la revendication 5, dans lequel la partie de traitement (10) est une boucle d'anse pour lier le tissu du corps.

9. Système d'endoscope comprenant :
un instrument de traitement endoscopique selon la revendication 1 ; et
un endoscope comprenant un canal (3) qui a une ouverture à son extrémité distale et dans lequel l'instrument de traitement endoscopique est reçu de façon à s'étendre et se rétracter à travers l'ouverture d'extrémité distale, et une unité de levée de forceps (5) qui est disposée au voisinage de l'extrémité distale du canal (3) et commande une direction de saillie de l'instrument de traitement endoscopique.

10. Système d'endoscope comprenant :
un instrument de traitement endoscopique selon la revendication 5 ; et
un endoscope comprenant un canal (3) qui a une ouverture à son extrémité distale et dans lequel l'instrument de traitement endoscopique est reçu de façon à s'étendre et se rétracter à travers l'ouverture d'extrémité distale, et une unité de levée de forceps (5) qui est disposée au voisinage de l'extrémité distale du canal (3) et commande une direction de saillie de l'instrument de traitement endoscopique.
